# EUROPEAN PATENT SPECIFICATION

(11) **EP 1 636 361 B1**
(45) Date of publication and mention of the grant of the patent: **17.06.2009**
(21) Application number: 03817327.4
(22) Date of filing: 26.06.2003
(51) Int. Cl.: C12N 15/10

(54) **METHOD FOR THE ISOLATION OF EXPRESSED SEQUENCE TAGS IN PLANTS**
VERFAHREN ZUR ISOLIERUNG EXPRIMIERTER SEQUENZ-TAGS IN PFLANZEN
PROCEDE D'ISOLATION D'ETIQUETTES DE SEQUENCES EXPRIMEES DANS DES PLANTES

(43) Date of publication of application: 22.03.2006
(73) Proprietor: Metapontum Agrobios S.r.l., 75010 Bernalda-Matera (IT)
(72) Inventor: CELLINI, Francesco, I-75100 Matera (IT); CIFARELLI, Rosa, Anna, I-75024 Montescaglioso-Matera (IT); GALLITELLI, Maria, I-75012 Bernalda-Matera (IT); MANGO, Teresa, I-87100 Cosenza (IT); LAURIA, Giuseppe, I-75100 Matera (IT); SEMERARO, Lucia, I-75100 Matera (IT)
(74) Representative: De Gregori, Antonella
(86) International application number: PCT/EP2003/006839
(87) International publication number: WO 2005/003344

(56) References cited:
- WO-A-96/36693
- SANO H ET AL: "A NOVEL RAS-RELATED RGP1 GENE ENCODING A GTP-BINDING PROTEIN HAS REDUCED EXPRESSION IN 5 AZACYTIDINE-INDUCED DWARF RICE" MOLECULAR & GENERAL GENETICS, vol. 228, no. 1-2, 1991, pages 227-232, XP002213260 ISSN: 0026-8925
- SANO H ET AL: "A SINGLE TREATMENT OF RICE SEEDLINGS WITH 5 AZACYTIDINE INDUCES HERITABLE DWARFISM AND UNDERMETHYLATION OF GENOMIC DNA" MOLECULAR & GENERAL GENETICS, vol. 220, no. 3, 1990, pages 441-447, XP002213261 ISSN: 0026-8925

## Description

The present invention relates to a method for the isolation of Expressed Sequence Tags (ESTs) which are based on the use of suitable quantities of 5-azaCytidine in the germination phase of plants to induce the demethylation phenomenon of the DNA. In this way, it is possible to obtain "totipotent" seedlings, i.e. capable of expressing as many genes as possible regardless of the development phase of the plant and isolating said genes by the synthesis of a single cDNA library.

The tern cDNA library means the combination of cDNA prepared from the whole population of mRNA of a tissue, a cellular line or an organism. It represents the collection of genes expressed in a cell or in a particular tissue, cloned in a suitable vector.

In order to obtain an overall picture of the genes which are expressed in the various development stages of an organism, it is necessary to prepare several libraries using mRNA extracted from the various tissues, or synthesize a single EST library using a messenger RNA pool extracted from different tissues.

These systems however involve an enormous amount of time, work and money.

The disadvantages of the known art mentioned above can be overcome by means of the method of the present invention which is based on the use of suitable quantities of 5-azaCytidine in the germination phase of plants to induce the DNA demethylation phenomenon. In this way, it is possible to obtain "totipotent" seedlings after a few weeks, i.e. capable of expressing as many genes as possible regardless of the development phase of the plant and isolating said genes by the synthesis of a single cDNA library.

The DNA methylation process is involved in various fundamental cellular events, such as, for example, embryogene development and genetic disorder. Furthermore, this process is considered as being one of the most important control mechanisms for genomic imprinting, see e.g. Sano, H. et al, Mol. Gen. Genet. (1990) 220: 441-447.

It intervenes in fact in the regulation processes of the gene expression by methylating the sections of DNA corresponding to the genes which must not be expressed in a certain tissue development phase. 5-AzaCytidine, a molecule which, if present during DNA duplication in the cells, can substitute cytosine, is not methylated by methyl transferase and the genome and all the subsequent genomes deriving from the hypomethylated helix remain hypomethylated. The inhibition of methyl transferase and hypomethylation of the DNA consequently favour a "totipotent" gene expression.

In accordance with this, the objective of the present invention relates to a method for the isolation of expressed sequence tags which are expressed in various development phases of plants which comprises the following steps:
(a) germination of seeds in a suitable medium in the presence of 5-azaCytidine in quantities ranging from 0.1 mM to 2 mM;
(b) extraction of the nucleic acids from the shoots grown as specified in step (a);
(c) synthesis of the cDNA library starting from the nucleic acids extracted in step (b);
(d) sequencing and sequence analysis of the clones of the library.

### Description of the figures

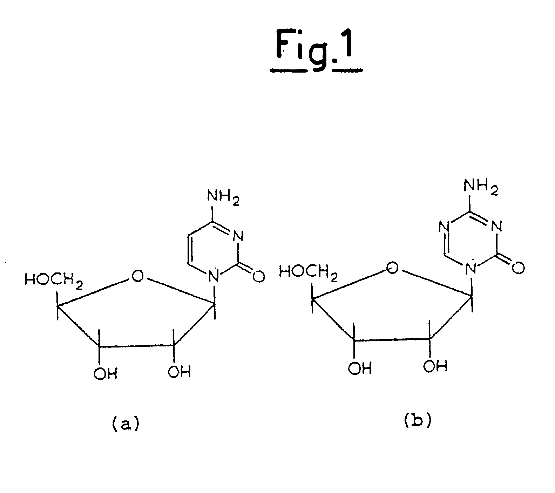

Figure 1: this shows the chemical structure of Cytosine (a) and 5-AzaCytidine (b).

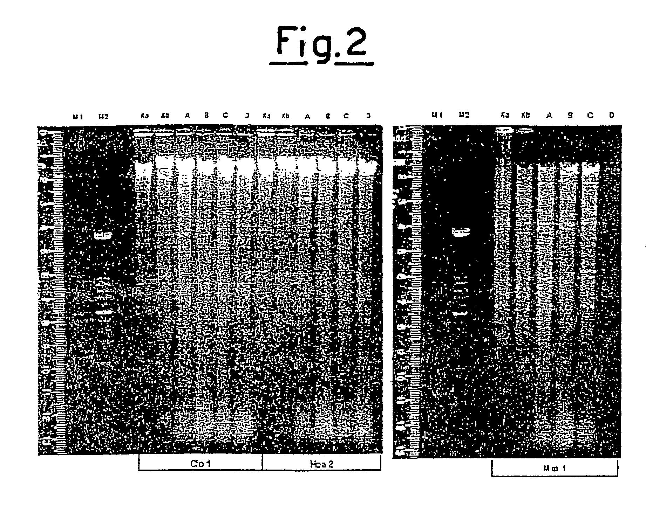

Figure 2: Digestion of the genomic DNA of hard corn, Ofanto variety, with restriction enzymes sensitive to methylation (CfoI, Hpa2, Mspl). Samples: Ka = DNA extracted from field leaves, the remaining samples are DNA extracted from seedlings germinated in a growth chamber on MSO medium containing 5-Aza-Cytidine at different concentrations: Kb = 0 mM, A = 0.1 mM, B = 0.3 mM, C = 0.5 mM and D = 1 mM.
   M1 and M2 are 2 molecular weight markers, 1 Kb (Gibco-BRL) and MXIV (Roche) respectively.

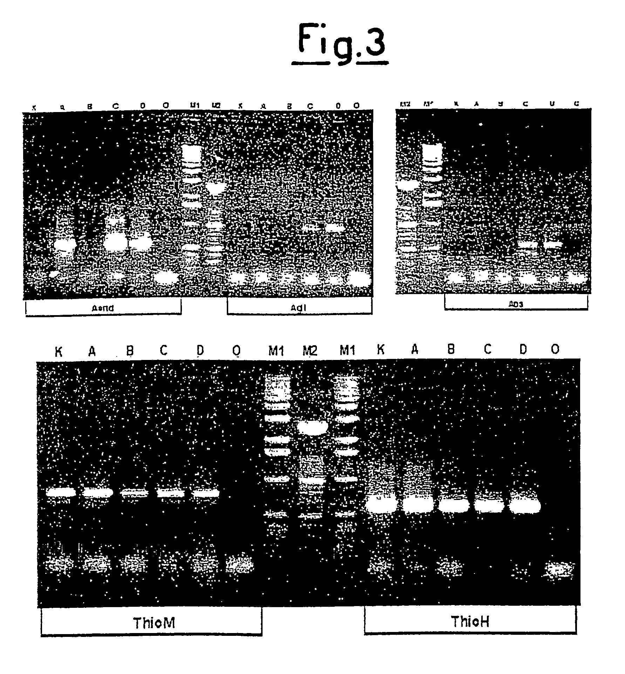

Figure 3: PCR Amplification on cDNA extracted from seedlings germinated in a growth chamber on MSO medium containing 5-Aza-Cytidine at different concentrations K = 0 mM, A = 0.1 mM, B = 0.3 mM, C = 0.5 mM and D = 1 mM. The abbreviations below indicate the pairs of primers used in the PCR reactions. Aend = EST from endosperm; Agli = gene encoding α-gliadin; APA = EST from precocious inflorescence phases; ThioM = ThioredoxinaM and ThioH = ThioredoxinaH. M1 and M2 are 2 molecular weight markers, 1 Kb (Gibco-BRL) and MXIV (Roche) respectively.

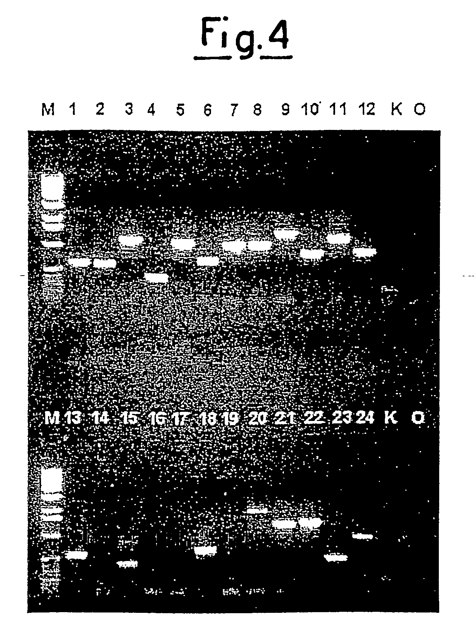

Figure 4: Amplification carried out on 24 phagic plaques selected at random to verify the size of the insert. K indicates the vector without an insert, M the molecular weight marker 1 Kb (Gibco-BRL).

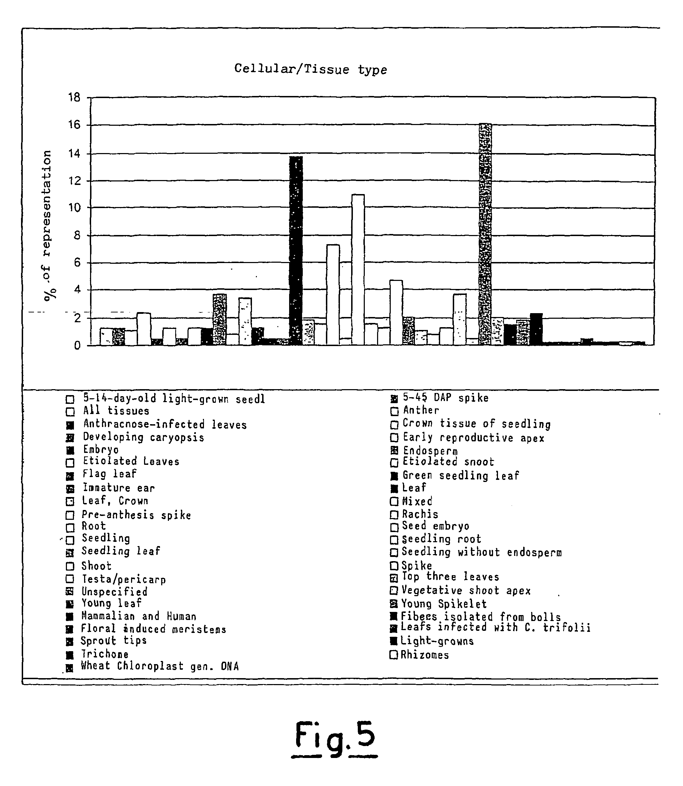

Figure 5: Summary graph of FASTAs relating to the sequence analyses carried out on some clones (385) of the "totipotent" EST libraries of hard corn. The graph indicates the representation percentages of each cellular type or tissue with which the best homology was found, for each clone.

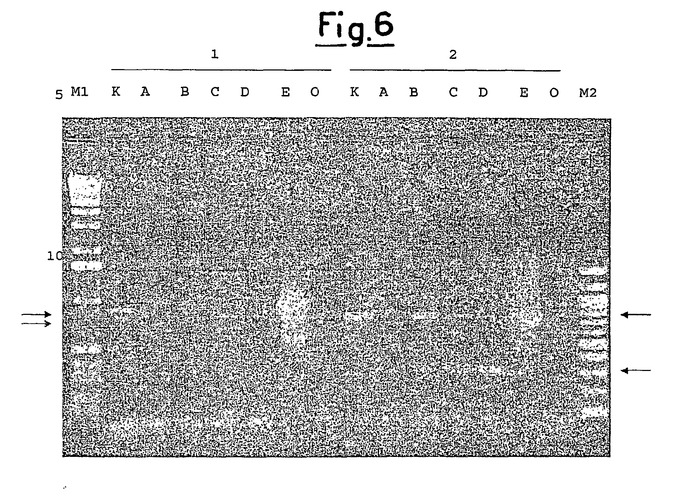

Figure 6: PCR amplification on cDNA extracted from seedlings germinated in a growth chamber on MSO medium containing 5-Aza-Cytidine at different concentrations K = 0 mM, A = 0.1 mM, B = 0.3 mM, C = 0.5 mM, D = 1 mM, E = genomic DNA of tomato plants, 0 = PCR mix without nucleic acids. M1 and M 2 are 2 molecular weight markers, 1Kb (Gibco-BRL) and MXIV (Roche) respectively.
   Whereas the red arrow indicates the fragment amplified by cDNA, the black arrow indicates the fragment amplified by the genomic DNA.
   1 = amplification with the primers specified for the gene of pollen LAT59
   2 = amplification with the primers specified for the gene of anthers LAT52.
Table 1: Summary table of FASTAs, relating to the sequence analyses carried out on some clones (50) of the EST library of hard corn. The table indicates, for each clone, the two sequences with the best homology. In some cases, a single sequence was inserted, if the homology percentage of the subsequent ones is lower than 60.0%.

### Detailed description of the invention

The method of the present invention is illustrated hereunder with reference to hard corn seeds, but can be used for any plant.

According to this method, the germination of the seeds is generally effected at a temperature ranging from 20 to 30°C, preferably from 22 to 26°C, in the dark and in the presence of concentrations of 5-azaC ranging from 0.1 mM to 2 mM, preferably from 0.3 mM to 0.5 mM.

The phenotype of the seedlings being tested, compared with the control phenotype of those cultivated without 5-azaC, is strictly correlated to the concentration of 5-azaC used, an increase in the concentration of 5-azaC corresponds to a slowing down in the development of the seedlings, with respect to both the aerial part and the roots.

Approximately 21 days after germination, the DNA, total RNA and messenger RNA were extracted from the seedlings.

Analyses for correlating the demethylation degree of the DNA with an increase in the undifferentiated gene expression were carried out by digestion of the genomic DNA with restriction enzymes sensitive to methylation, i.e. enzymes which preferentially act on hypomethylated DNA.

On the basis of the results obtained, a different digestion of the genomic DNA is observed, in relation to the concentrations of 5-azaC adapted.

Furthermore, to confirm the efficiency of the method used, several genes were identified which are expressed in different growth phases of soft corn, of which a large number of sequences are available as this species of corn is more widely studied than hard corn. On the basis of the sequences deposited in data banks (National Center for Biotechnology Information, NCBI; European Bioinformatics Institute, EBI) pairs of oligonucleotides identified in the zones flanking the region encoding the nucleotidic sequence, were prepared, and subsequently used as primers for the amplification of the whole gene being tested.

The name and membership tissue of the genes selected are listed below:
- Aesend, EST from immature endosperm;
- Aesgliad, gene which encodes α-gliadin in the seeds;
- Aespre-Ant, EST which is expressed in precocious inflorescence phases;
- Thiom, gene which encodes thioredoxinaM in all the tissues;
- Thioh, gene which encodes thioredoxinaH in all the tissues.

Each expressed sequence tag was isolated using the polymerase chain reaction technique (RT-PCR) on the total RNA with a pair of oligonucleotides flanking the encoding region.

The cDNA inserts were first amplified with the appropriate primers, cloned in the vector p-GEMT and introduced into the competent cells Escherichia coli (E.coli) DH5α. The recombinant clones, containing the expected fragments, were characterized by restriction analysis and their identity was confirmed by effecting sequence reactions carried out using the ABI Prism Big TaqDyeDeoxyTerminator Cycle Sequencing Kit (Applied Biosystems, Nr. 4303149) and analyzed with the automatic ABI 377 DNA Sequencer (Perkin Elmer ABI Prism).

The nucleotidic sequences obtained were compared with those deposited in public data banks (NCBI, EBI). An analysis of the data confirmed the efficiency of the method, demonstrating that the best results are obtained with a concentration of 5-AzaCytidine in the germination medium equal to 0.3-0.5 mM.

A cDNA expression library from plants of Triticum turgidum germinated in MSO medium containing 0.3 mM of 5-AzaCytidine, was subsequently prepared, in the phagic vector lambda Uni-ZAP XR. Polyadenylated messenger RNA was used for the synthesis of the double-strand cDNA operating according to the procedures suggested by the kit distributing company (STRATAGENE).

The molecules of DNA with a high molecular weight used for constructing the library were separated from those with a low molecular weight which represent the fraction of molecules in which the synthesis was not completed.

The fraction of cDNA corresponding to the high molecular weight fraction was inserted in the phagic vector lambda Uni-ZAP XR and packed with the packaging extracts containing proteins for the head and tail of the phage. The total quantity of phagic particles obtained from the packaging in vitro was determined by plating small aliquots with the host bacterial strain XL1-Blue MRF'.

The dimensions of the inserts, present in the library produced, were verified by subjecting various phagic plaques selected at random to amplification reaction and using a specific pair of primers for the vector Uni-ZAP XR.

The results obtained showed that the fragments of the primary cDNA library have an average dimension ranging from 0.5 to 1.6 Kb.

For the amplification of the primary library, the phages were used to infect the host cells XL1 Blue MRF' which, by allowing the replication of the phages in their inside and following their lysis, enabled a library consisting of about 1 x 10⁹ phagic particles per ml, to be recovered.

After amplification, the library in the lambda phage was converted to a plasmidic library by means of total excision in vivo. The vector Uni-ZAP XR was prepared so as to allow an efficient excision in vivo of the inserts cloned in the lambda vector to form phagemid.

Excision in vivo depends on particular DNA sequences present in the vector and with different proteins, including those proteins deriving from the helper phage, and is favoured by SLOR cells which, due to their characteristics, eliminate problems associated with co-infection with the helper phage, by inhibiting it.

SLOR cells of the strain E.coli were therefore transformed with phagemids, plated on selective medium for pBluescriptsk (+/-) phagemid containing ampicillin to form colonies. The titer of the library in excised phagemid is 1.5 x 10¹² colonies per ml.

The DNA extracted from the colonies was then used for sequence analysis.

The results of the sequencing of EST clones and their comparison with data banks showed homologous sequences with different tissues (leaves, ripe seeds, flowers and roots) even though the seedlings were only 21 days old. As can be observed in figure 5, in fact, homologies can be seen with genes involved in the starch metabolism, genes encoding reserve proteins, which are expressed in the roots or inflorescence phases. As expected for a typical cDNA library, 20% of the EST clones sequenced did not show any homology with sequences whose function is known.

The following examples are illustrative but do not limit the scope of the invention described.

The method of the invention can usually be applied in kits for the synthesis of totipotent cDNA libraries. In practice, the new kit should also comprise, in addition to the kit components currently on the market (as described for example in Table 1 of the handbook for the Library Construction of STRATAGENE Catalogue Nr. S200450), the necessary components for the embodiment of the method of the invention, such as:
a solution of 5-AzaCytidine;
- tomato and/or wheat seeds germinated in a suitable medium in the presence of 5-Azacytidine in quantities ranging from 0,1 mM to 2 mM,
- primer pairs for the amplification of genes expressed in different growth phases of tomoto and soft corn;
- description of the procedure for the embodiment of the method of the invention.

### EXAMPLE 1

### Extraction of the genomic DNA and messenger RNA

Seeds of hard corn (Triticum durum) were sterilized in a solution of ethyl alcohol at 70% for 10 minutes under stirring. After eliminating the ethanol, the seeds were treated with a solution containing sodium hypochlorite (ACE) at 50% and Sodium Dodecyl Sulfate (SDS) at 0.5%, incubated at room temperature for 20 minutes under light stirring.

The seeds were subsequently washed with sterile H₂O until the complete removal of the foam (about 7-8 times) and about 15-20 seeds were then placed in a Magenta Box each containing: 30 ml of MSO substrate (Sucrose 15 g/l, Muraschige-Sckug MS salts 2.2 g/l, Thiamin 0.2 mg/l, Myoinositol 50 mg/l, pH 5.6, agar 7 g/l), different concentrations of 5-azac (0.1 mM, 0.3 mM, 0.5 mM and 1 mM) and germinated in a chamber thermostat-regulated at 24°C in the dark.

Seeds of hard corn left to germinate in MSO medium without 5-azac, were used as a control.

After about 21 days of germination, the DNA, total RNA and messenger RNA were extracted from the seedlings using the Invitrogen kit (Fasttrack^{™} 2.0 Kit, Nr. K1593-02, K1593-03).

Analyses for correlating the demethylation degree of the DNA with an increase in the undifferentiated gene expression were carried out by digesting the genomic DNA with the following restriction enzymes: Cfol, Hpa2, Msp1 (Roche), sensitive to methylation.

For each reaction, about 5 µg of total DNA were digested with 20 units of enzyme in a final volume of 150 µl, for a night at 37°C. Each digestion product was precipitated and resuspended in 30 µl of H₂O. The digestion mixtures were separated on agarose gel at 0.8% and subjected to horizontal electrophoresis, for a night at 25 mV (Sambrook, J. et al., 1989, Cold Spring Harbor Laboratory Press).

In all cases differences were observed with respect to the control, which indicate a different methylation of the genome analyzed (Figure 2).

Furthermore, in order to confirm the efficiency of the method used, some of the genes identified in literature which are expressed in different growth phases of soft corn, were isolated.

About 3 µg of total RNA were used for the synthesis of double-strand cDNA using the kit distributed by PHARMACIA (catalogue nr.: 27-9260-01). The experimental conditions used were those suggested by the distributing company of the kit.

A pair of nucleotides flanking the encoding region was prepared for each gene. The name, size and pair of primers in the direction 5'→3', are listed below:
- Aesend 588 bp; EST from endosperm; Accession number: BE401963;
   5' GGATCCTTCCAGAGTACCTG 3' (FORWARD, EndFor)
   5' TCTAGATAGCACTACCTACAAACAC 3' (REVERSE, EndRev).
- Aesgliad 1092 bp; alpha gliadin gene; Accession number; U08287;
   5' GGATCCGGTCAATACAAATCC 3' (FORWARD, GliaFor)
   5' AAGCTTCACCGCTACAACGACC 3' (REVERSE, GliaRev).
- Aespre-Ant 566bp; EST from pre-anthesis spike; Accession number: BES00795;
   5' GGATCCAAACGGCGCCCG 3' (FORWARD, PAntSFor)
   5' TCTAGATTTACTGCACTAGGAC 3' (REVERSE, PAntSRev).
- Thiom 781 bp; thioredoxina M; Accession number: AJ005840;
   5' GGATCCCTCCCTCTGTCTCC 3' (FORWARD, ThioMFor)
   5' TCTAGACGAACATGCATGTATACTG 3' (REVERSE, ThioMRev).
- Thioh 630 bp; thioredoxina H; Accession number: AJ001903.
   5' GGATCCCGTGAGAAATAAGCG 3' (FORWARD, ThioHFor)
   5' TCTAGATGAAATCAACCATTTACCG 3' (REVERSE, ThioHRev).

These oligonucleotides were used for the isolation of the corresponding fragments by means of the polymerase chain reaction technique (PCR).

The amplification was carried out in a GeneAmpPCRSystems9700^{R} thermal cycler (PE AppliedBioSystems) using, for each reaction, a mixture (25 µl) containing 6 µl of double-strand cDNA, 10 mM Tris-HCl pH 8.3, 50 mM KCl, 1.5 mM MgCl₂, 2.5 µM of each primer, 0.1 mM of dNTP and 2.5 Units of Taq DNA polymerase (Roche).

After a first denaturation cycle for 5 minutes at 95°C, the reaction continued with the following cycles:
1 minute at 94°C (denaturation)
1 minute at 56°C (pairing)
2 minutes at 72°C (elongation)
for a total of 35 cycles, followed by 10 minutes at 72°C (final extension).

The amplification were separated on agarose gel at 1%; the DNA bands of interest were recovered and purified with the GeneClean^{™} kit (BIO 101 Inc., USA, Nr. 1001-400).

About 100 ng of the DNA thus isolated, for each amplification product, were ligated to 50 ng of pGEM-T plasmid (Promega, Nr. A3600) in 10 µl of reaction mixture, in the presence of 2 units of T4 DNA ligase (Promega, Nr. A3600) and incubated at 4°C for a night.

2 µl of each mixture were used to transform competent cells of E.coli DH5α (BRL, Nr. 18258-012).

The recombinant clones were selected on Petri plates of LB solid medium (NaCl 10 g/l, yeast extracts 5 g/l, Bacto-triptone 10 g/l and agar 20 g/l) containing 100 mg/l of ampicillin.

6 clones were identified for each transformation event, from which the plasmid DNA, adopted for the sequence analyses, was extracted. The reactions and sequence analyses were carried out with the ABI Prism Big TaqDyeDeoxyTerminator Cycle Sequencing kit (Applied Biosystems, Nr. 4303149), using the GeneAmpPCRSystem9700^{R} (PE Applied BioSystems) as thermal cycler and the ABI Prism 377 DNA Sequencer (Applied Biosystems) as sequencer.

The sequence analyses carried out for each gene showed homology with the sequences of the genes selected, thus confirming the efficiency of the method used.

### EXAMPLE 2

### Construction of the cDNA hard corn library

The messenger RNA was extracted from etiolated seedlings of hard corn, Ofanto variety, germinated on MSO medium containing 5-AzaCytidine 0.3 mM, using the Invitrogen kit (Fasttrack^{TN}) 2.0 Kit, Nr. K1593-02, K1593-03).

The experimental conditions suggested by the kit distributor (Library Construction Kit, Stratagene, Catalogue Nr. S200450) were used for the synthesis of the EST (Expressed Sequence Tags) library.

About 5 µg of polyadenylated messenger RNA of hard corn were used for the synthesis of double-strand cDNA.

The end of the cDNA molecules were flattened by the action of DNA polymerase Pfu (5 Units, Stratagene), containing 3.6 µg of linkers having the restriction site EcoRI and subjected to digestion with the enzyme XhoI (120 Units) whose site is present in the polydT primer used for the synthesis of the first strand of the cDNA.

This gives rise to molecules having the EcoRI site at one end and the XhoI site at the other.

In order to separate molecules of cDNA with a high molecular weight, useful for constructing the library, from those having a low molecular weight represented by the fraction of molecule in which the synthesis had not been completed, the cDNA sample of Triticum durum was passed on a Sephacryl^{R} S-500 column, equilibrated in 20 mM of Tris-HCl pH 7.5, 10 mM EDTA, 100 mM of NaCl, and subjected to centrifugation for 2 minutes at 400 x g.

Three fractions of the library were recovered and their molecular weight was subsequently verified by the separation of an aliquot of each fraction on non-denaturing polyacrylamide gel at 5% (Sambrook, J. et al. (1989), Cold Spring Harbor Laboratory Press).

About 100 ng of the first fraction of cDNA recovered, corresponding to the high molecular weight fraction, were directionally ligated with 1 µg of the lambda phagic vector Uni-ZAP XR, pre-digested with EcoRI and XhoI, and packed with the packaging extracts containing proteins for the head and tail of the phage.

The total quantity of phagic particles obtained from the packing in vitro was determined by plating small aliquots with the host bacterial strain XL1-Blue MRF', i.e. by effecting its titer.

The primary library obtained contains a total of 2.3 x 10⁶ units forming plaques (pfu) per µg of arms of the ligated vector and 97% of these contain the DNA insert.

The following controls were effected parallelly:
(1) verification of the ligase efficiency using the vector pBR322 suitably linearized (supplied with the kit), as insert to be ligated to the phagic vector;
(2) verification of the packaging efficiency in which an aliquot of lambda DNA was packed and finally;
(3) control for monitoring the library background (non-recombinant clones) in which a ligase was effected, and consequently the packaging of the phagic vector alone.

The dimension of the library produced was verified by subjecting 24 phagic plaques selected at random and amplified with a pair of specific primers for the vector Uni-ZAP XR, to PCR reaction.
a) Primer Forward: 5' GTAAAACGACGGCCAGT 3' (pBSKFor);
b) Primer Reverse: 5' GGAAACAGCTATGACCATG 3' (pBSKRev).

The results obtained showed that the inserts of the cDNA library had an average dimension of 0.8 Kb (Figure 4)

### EXAMPLE 3

### Conversion of the phagic library Uni-ZAP XR to a plasmid library

The primary corn library was subsequently amplified to make it more stable and converted into a phagemid, in the phagemid vector pBluescriptSK of 2958 bp, by means of total excision in vivo according to the method described by the distributor of the kit adopted (Library Construction Kit, Stratagene, Nr. S200450).

More specifically, the host cells XL1 Blue MRF' were diluted to OD₆₀₀ 0.5 in 10 mM of MgSO₄. To amplify 1 x 10⁶ plaques, 20 aliquots of library were used, each aliquot contains 5 x 10⁴ plaques (11 µl) with which 600 µl of host cells were infected, and each aliquot was distributed on 150 mm plates containing NZY medium (NaCl 5 g/l, MgSO₄ 2 g/l, yeast extracts 5 g/l, NZ amines, hydrolyzed casein 10 g/l and agar 15 g/l, pH 7.5), incubated for 8 hours at 37°C.

The following day 10 ml of SM buffer (NaCl 5.8 g/l, MgSO₄ 2 g/l, Tris-HCl 1M, pH 7.5, 50 ml, gelatin 2% 5 ml) were added to each plate and incubated for the whole night at 4°C under stirring.

Once the phagic suspension had been recovered, the titer of the library was controlled again, and proved to be equal to about 7 x 10⁹ phagic particles per ml. To preserve the phagic library, the suspension recovered (40 ml) was divided into aliquots and distributed in 1 ml tubes, 0.3% of chloroform was added to a part of the aliquots (20), and preserved at 4°C, 7% of DiMethylSulfoxide was added to the remaining part (20) and preserved at -80°C.

In order to effect the excision of the library, a lambda phage/XL1 Blue MRF' cells ratio equal to 1:10 was used, and a helper phage/XL1 Blue MRF' cells ratio equal to 10:1.

In practice, 100 µl of the amplified library, corresponding to about 1 x 10⁸ phagic particles, were incubated with 1 x 10⁹ cells of XL1 Blue MRF', i.e. about 8 ml, and with 1 ml of ExAssist helper phage, corresponding to 1 x 10¹⁰ pfu, to generate phagemid particles containing the plasmid vector excised from the phagic vector.

The excess number of helper phages and E.coli cells, with respect to the number of phages of the library, was used to ensure that each cell was infected both by the helper phages and by the lambda phage in order to obtain an efficient and representative excision in vivo.

The incubation of the lambda phages with the helper phages and XL1 Blue MRF' cells took place fro 15 minutes at 37°C, after which 20 ml of LB medium were added and the incubation was continued at 37°C for a further 3 hours.

In order to lyse the phagic particles and allow the release and recovery of the phagemid particles, the suspension was incubated at 70°C for 20 minutes and then centrifuged for 10 minutes at 500 x g. The surnatant was recovered and preserved at 4°C.

1 x 10⁸ phagemids (1 µl of the surnatant) were subsequently incubated with 200 µl of SLOR E.coli cells (ratio 10:1), at 37°C for 15 minutes, in order to obtain SLOR bacterial cells containing the hard corn EST library in a plasmid vector.

3 tests were then carried out, by plating 100 µl, 10 µl and 1 µl on solid LB medium (NaCl 10 g/l, yeast extracts 5 g/l, Bacto-triptone 10 g/l and agar 2.0 g/l) containing ampicillin at a concentration of 100 mg/l; the plates were incubated at 37°C for 8 hours.

The titer of the excised phagemid library is 1.5 x 10¹² colonies per ml. The phagemid library was subsequently multiplied in the strain of E.coli SLOR and the clones obtained were distributed in plates with 96 cavities.

The preservation medium is the "Cell Freezer Storage Medium", consisting of LB (NaCl 10 g/l, yeast extracts 5 g/l, Bacto-triptone 10 g/l), containing 100 mg/l of ampicillin and agar 20 g/l; 1 X Freezer Buffer (K₂HPO₄ 62.7 g/l, KH₂PO₄ 18 g/l, Na Citrate 5 g/l, MgSO₄ 1 g/l, (NH₄)₂SO₄ 9 g/l, Glycerol 440 ml). Each colony was transferred to a cavity containing 1 ml of the above solution containing 0.1 ml of LB with ampicillin. The plates were incubated for a night at 37°C and then preserved at -80°C.

### EXAMPLE 4

### EST library screening, sequence analysis and comparison with data banks

In order to effect the analysis of the expressed sequence tags (EST) in the hard corn cDNA library, the plasmid DNA was extracted from the plates with 96 cavities and subjected to sequence analysis.

An aliquot (3 µl) taken from each single cavity, containing a clone, was incubated for a night at 37°C, in 1.2 ml of LB with ampicillin. The purification of the DNA was carried out using BIOMECK2000 (Beckman), an automatic liquid handling station, and the extraction kit of Promega, (Wizard SV96, Plasmid DNA Purification System Nr. A2255), effecting all the operations indicated by the manufacturer.

The quantification of the extracted DNA was then effected, charging 5 µl on agarose gel at 1%.

Each clone was subjected to sequence reaction, using universal oligonucleotides as primers, which can be found in the polylinker of the pBluescriptsk vector in both directions:
- Primer Forward: 5' GTAAAACGACGGCCAGT 3' (pBSKFor);
- Primer Reverse: 5' GGAAACAGCTATGACCATG 3' (pBSKRev).

About 350 ng of plasmid DNA were used for each sequence reaction, following the procedures suggested by the protocol of the ABI Prism Big TaqDyeDeoxyTerminator Cycle Sequencing kit (Applied Biosystems, Nr. 4303149).

The amplifications were carried out using the equipment for PCR GeneAmp PCR System 9700 (PE-Applied Biosystems). The reaction products were charged on LongRanger Single pack, denaturing polyacrylamide gel, type 377 (Biowhittaker Molecular Applications, Nr. 50691), and the electrophoresis run was carried out using the automatic sequencer ABI 377 DNA Sequencer (Applied Biosystems).

The sequence analyses were carried out using the Sequencer^{™} program (Gene Codes Corporation).

The data obtained for each clone were compared with the sequences deposited in public data banks, such as FASTA and BLAST (National Center for Biotechnology Information, NCBI; European Bioinformatics Institute, EBI).

An example of the results obtained is indicated in Table 1 and in figure 5.

**Table 1**

| Clone | Insert (bp) | Accession number | Homology (%) | Organism | Cellular type/ tissue |
|---|---|---|---|---|---|
| cDNA 00010 | 875 | BE060569 | 82.8 | Hordeum vulgare | Spike before flowering |
| | | AP003436 | 78.7 | Oryza sativa | Non-specified tissue |
| cDNA 00012R | 620 | AP003934 | 81.0 | Oryza sativa | Non-specified tissue |
| | | AP003722 | 81.0 | Oryza sativa | Non-specified tissue |
| cDNA 00013 | 354 | AI834373 | 62.0 | Zea Mays | Unripe spike |
| cDNA 00014 | 759 | BE587421 | 95.3 | Secale cereale | Root end |
| | | AW564255 | 68.7 | Sorghum bicolor | Seedling grown in the light |
| cDNA 00015 | 394 | BE405857 | 81.9 | Triticum aestivum | Root |
| | | BE637241 | 85.5 | Secale cereale | Anther |
| cDNA 00016 | 904 | BG418106 | 89.0 | Hordeum vulgare | Head/pericarp |
| | | BG873973 | 79.0 | Zea mays | Young seedling |
| cDNA 00017 | 915 | BF482801 | 98.7 | Triticum aestivum | Spike before flowering |
| | | BF277210 | 67.4 | Gossypium arboreum | Fibres isolated from capsule |
| cDNA 00018 | 693 | BG905579 | 98.4 | Triticum aestivum | Leaves |
| | | BE216980 | 92.2 | Triticum aestivum | Leaves |
| cDNA 00019 | 1011 | BG418804 | 89.0 | Hordeum vulgare | Head/pericarp |
| | | BE606987 | 95.9 | Triticum aestivum | Spike |
| cDNA 00020R | 598 | X56882 | 89.4 | Triticum aestivum | Embryonal axis |
| | | BE471153 | 91.1 | Triticum aestivum | Young seedling without endosperm |
| cDNA 00021 | 724 | BE429737 | 94.2 | Triticum aestivum | Non-specified tissue |
| | | BE426779 | 94.2 | Triticum aestivum | Etiolated shoot |
| cDNA 00022R | 595 | BG414796 | 85.3 | Hordeum vulgare | Head/pericarp |
| | | BG907822 | 98.17 | Triticum aestivum | Leaves |
| cDNA 00024R | 569 | BE366369 | 81.6 | Sorghum bicolor | Leaves with anthracnose |
| | | BG102688 | 81.6 | Sorghum propinquum | Rhizomes |
| cDNA 00025F | 616 | BE213392 | 95.8 | Triticum aestivum | Leaves |
| | | BE490543 | 96.4 | Triticum aestivum | Young seedling |
| cDNA 00034F | 628 | BE427302 | 87.4 | Triticum aestivum | Head/pericarp |
| | | BE426779 | 96.2 | Triticum aestivum | Spike before flowering |
| cDNA 00046F | 202 | BF200965 | 87.5 | Triticum aestivum | Tissue of seedling crown |
| | | BE488428 | 81.7 | Triticum aestivum | Etiolated shoot |
| cDNA 00050F | 532 | BE404845 | 92.7 | Triticum aestivum | Root |
| | | BE429889 | 94.3 | Triticum aestivum | Non-specified tissue |

### EXAMPLE 5

### Verification of the method in tomato plants

Tomato seeds (Lycopersicon esculentum, cv. Red Setter) were sterilized in a solution of ethyl alcohol at 70% for 10 minutes under stirring. After eliminating the ethanol, the seeds were treated with a solution containing sodium hypochlorite (ACE) at 50% and Sodium Dodecyl Sulfate (SDS) at 0.5%, incubated at room temperature for 20 minutes, under stirring.

The seeds were subsequently washed with sterile H₂O until the foam had been completely removed (about 7-8 times) and about 15-20 seeds were then positioned in a Magenta Box, each containing: 30 ml of MSO substrate (Sucrose 15 g/l, MS Muraschigo-Sckug salts 2.2 g/l, Thiamine 0.2 mg/l, Myoinositol 50 mg/l, pH 5.6, agar 7 g/l), different concentrations of 5-azac (0.1 mM, 0.3 mM, 0.5 mM and 1 mM) and germinated in a dark room thermostat-regulated at 24°C.

Tomato seeds left to germinate in MSO medium without 5-azac, were used as a control.

After about 21 days of germination, the total RNA was extracted from the etiolated seedlings, using the Invitrogen kit (Fasttrack^{™} 2.0 Kit, Nr. K1593-02).

In order to confirm the efficacy of the method used, some of the genes identified in literature, which are expressed in different growth phases of tomato plants, were isolated.

About 3 µg of total RNA were used for the synthesis of double-strand cDNA using the kit distributed by PHARMACIA (catalogue Nr: 27-9260-01). The experimental conditions adopted were those suggested by the kit supplier.

A pair of oligonucleotides flanking the codifying region was designed for each gene. The name, size and pair of oligonucleotides in the direction 5'→3', are provided below:
- LAT59 1350bp; gene which is expressed, in pollen; Accession number: X15499;
   5' CAAAGGAGCCATTGTGGAT 3' (FORWARD, LAT59-For)
   5' GCTGGAGCTGCTGATATTCC 3' (REVERSE, LAT59-Rev)
- LAT52 486bp; gene which is expressed in anthers; Accession number: X15855;
   5' ATGGCAAAGGCTATTGTGCT 3' (FORWARD, LAT52-For)
   5' CTCTTTGCAGTCCTCCCTTG 3' (REVERSE, LAT52-Rev).

These oligonucleotides were used for the isolation of the corresponding fragments by means of the polymerase chain reaction (PCR) technique.

The amplification was effected in a GeneAmpPCRSystem9700^{R} thermocycler (PE AppliedBioSystems) using, for each reaction, a mixture (25 µl) containing 6 µl of double-strand cDNA, 10 mM of Tris-HCl pH 8.3, 50 mM of KCl, 1.5 mM of MgCl₂, 2.5 µm of each primer, 0.1 mM of dNTP and 2.5 Units of Taq DNA polymerase (Roche).

After a first denaturation cycle at 95°C for 5 minutes, the reaction continued with the following cycles:
1 minute at 94°C (denaturation),
1 minute at 56°C (pairing)
2 minutes at 72°C (elongation)
for a total of 35 cycles followed by 10 minutes at 72°C (final extension).

The amplification products were separated on agarose gel 1%, the DNA bands of interest were recovered and purified with a GeneClean^{™} kit (Bio 101 Inc., USA, Nr. 1001-400).

About 100 ng of the DNA thus isolated, for each amplification product, was used for the sequence analyses. The reactions and sequence analyses were carried out with an ABI Prism Big TaqDyeDeoxyTerminator Cycle Sequencing kit (Applied Biosystems, Nr. 4303149), using a GeneAmpPCRSystem9700R as thermocycler (PE AppliedBioSystems) and, as sequencer, an ABI Prism 377 DNA Sequencer (Applied Biosystems).

The sequence analyses carried out for each gene showed complete similarity with the sequences of the genes selected, thus confirming the efficacy of the method used.

## Claims

1. A method for the isolation of expressed sequence tags in different development phases of plants which comprises the following steps:
(a) germination of the seeds in a suitable medium in the presence of 5-azaCytidine in quantities ranging from 0.1 mM to 2 mM;
(b) extraction of the nucleic acids from the shoots grown as specified in step (a);
(c) synthesis of the cDNA library starting from the nucleic acids extracted in step(b);
(d) sequencing and sequence analysis of the clones of the library.

2. The method according to claim 1, wherein said step (a) of germination of seeds has a duration of 21 days.

3. The method according to claim 1, wherein the quantity of azaCytidine in step (a) ranges from 0.3 mM to 0.5 mM.

4. The method according to claim 1, wherein in step (a) the germination is carried out at a temperature ranging from 20 to 30°C.

5. The method according to claim 4, wherein the temperature ranges from 22 to 26°C.

6. A kit for the synthesis of cDNA libraries of plants comprising the necessary components and instructions for the embodiment of the method according to claim 1, said components and instructions comprising:
- a solution of 5-Azacytidine;
- tomato and/or wheat seeds germinated in the dark in a suitable medium in the presence of 5-Azacytidine in quantities ranging from 0.1 mM to 2 mM, preferably from 0.3 mM to 0.5 mM;
- primer pairs for the amplification of genes expressed in different growth phases of tomato and soft corn;
- a description of the procedure for the embodiment of the method of claim 1.

## Patentansprüche

1. Verfahren zum Isolieren von exprimierten Sequenzanhängseln in verschiedenen Entwicklungsphasen von Pflanzen, welches die folgenden Stufen umfasst:
(a) Keimung der Samen in einem geeigneten Medium in Anwesenheit von 5-Azacytidin in einer Menge im Bereich von 0,1 mM bis 2 mM;
(b) Extrahieren der Nucleinsäuren aus den in Stufe (a) gewachsenen Trieben;
(c) Synthetisieren der cDNA-Bibliothek beginnend mit den in Stufe (b) extrahierten Nucleinsäuren;
(d) Sequenzieren und Analysieren der Sequenz der Klone der Bibliothek.

2. Verfahren nach Anspruch 1, wobei die Stufe (a) des Keimens der Samen eine Dauer von 21 Tagen umfasst.

3. Verfahren nach Anspruch 1, wobei die Menge von Azacytidin in Stufe (a) im Bereich von 0,3 mM bis 0,5 mM ist.

4. Verfahren nach Anspruch 1, wobei in Stufe (a) die Keimung bei einer Temperatur im Bereich von 20 bis 30 °C durchgeführt wird.

5. Verfahren nach Anspruch 4, wobei die Temperatur im Bereich von 22 bis 26 °C liegt.

6. Kit für die Synthese von cDNA-Bibliotheken von Pflanzen, welcher die notwendigen Bestandteile und Anweisungen für die Ausführung des Verfahrens nach Anspruch 1 enthält, wobei die Komponenten und Anweisungen folgendes umfassen:
- eine Lösung von 5-Azacytidin;
- im Dunkeln in einem geeigneten Medium in Anwesenheit von 5-Azacytidin gekeimte Tomaten- und/oder Weizensamen in einer Menge im Bereich von 0,1 mM bis 2 mM, vorzugsweise 0,3 mM bis 0,5 mM;
- Primerpaare für die Amplifikation von in unterschiedlichen Wachstumsphasen von Tomaten und Weichkorn exprimierten Genen;
- eine Beschreibung der Vorgehensweise zum Ausführen des Verfahrens nach Anspruch 1.

## Revendications

1. Procédé d'isolement de marqueurs de séquence exprimée à différentes phases de développement de végétaux qui comprend les étapes suivantes :
(a) germination des graines dans un milieu approprié en présence de 5-azacytidine en quantités allant de 0,1 mM à 2 mM ;
(b) extraction des acides nucléiques des pousses cultivées comme indiqué à l'étape (a) ;
(c) synthèse de la banque d'ADNc à partir des acides nucléiques extraits à l'étape (b) ;
(d) séquencement et analyse de séquence des clones de la banque.

2. Procédé selon la revendication 1, dans lequel ladite étape (a) de germination des graines dure 21 jours.

3. Procédé selon la revendication 1, dans lequel la quantité d'azacytidine de l'étape (a) va de 0,3 mM à 0,5 mM.

4. Procédé selon la revendication 1, dans lequel à l'étape (a) la germination est réalisée à une température allant de 20°C à 30°C.

5. Procédé selon la revendication 4, dans lequel la température va de 22°C à 26°C.

6. Trousse pour la synthèse de banques d'ADNc de végétaux comprenant les composants et instructions nécessaires pour la réalisation du procédé selon la revendication 1, lesdits composants et instructions comprenant :
- une solution de 5-azacytidine ;
- des graines de tomate et/ou de blé ayant germé dans l'obscurité dans un milieu approprié en présence de 5-azacytidine en quantités allant de 0,1 mM à 2 mM, de préférence de 0,3 mM à 0,5 mM ;
- des paires d'amorces pour l'amplification des gènes exprimés lors des différentes phases de croissance de la tomate et du maïs tendre ;
- une description du protocole de réalisation du procédé selon la revendication 1.
